# EUROPEAN PATENT APPLICATION

(11) **EP 2 193 773 A1**
(43) Date of publication of application: **09.06.2010**
(21) Application number: 09179638.3
(22) Date of filing: 29.06.2007
(51) Int. Cl.: A61F 13/15

(54) **Disposable article with serviceable indicia**

(30) Priority: 30.06.2006 US 817839 P
(62) Divisional of application: 07789840.1
(71) Applicant: The Procter and Gamble Company, Cincinnati, Ohio 45202 (US)
(72) Inventor: Mc Cusker III, Henry William, 1080, Caracas (VE); Roe, Donald, West Chester, OH 45069 (US); Fuchs, Christofer, 65760, Eschborn (DE); Kline, Mark James, Okeana, OH 45053 (US); Tausch, Jennifer Lyn, Cincinnati, OH 45252 (US)
(74) Representative: McGregor, Judit Ester

(57) **Abstract**

A disposable absorbent article to be worn about the lower torso of a wearer that facilitates an easy, intuitive change is provided. The disposable article includes a serviceable indicium that facilitates fitting of the disposable absorbent article to the wearer. The serviceable indicium aids in aligning the disposable absorbent article to the wearer and provides guidance on how to grasp the disposable absorbent article during fitting of the disposable absorbent article to the wearer.

## Description

### FIELD OF THE INVENTION

The invention relates generally to hygienic absorbent articles, and, more specifically, to absorbent articles configured to facilitate the process of fitting the diaper to a wearer.

### BACKGROUND OF THE INVENTION

Absorbent articles, such as diapers, training pants, and the like, are well known in the art. These articles typically have an absorbent core held or positioned in proximity to the body of a wearer during use by a fastening system in order to capture and absorb bodily exudates discharged from the wearer. Typical absorbent articles include a topsheet facing the wearer, which permits fluid exudates to pass through, and a backsheet, which prevents the exudates from escaping from the absorbent article.

Disposable absorbent articles such as diapers are designed to absorb and contain bodily waste in order to prevent soiling of the body and clothing of the wearer. The disposable diapers generally comprise a single design available in different sizes to fit a variety of wearers ranging from newborns to toddlers undergoing toilet training. The design and fit of the diaper can affect performance, such as the ability to absorb and contain bodily waste. The size of the diaper further can affect fit, for example, the size of the diaper waist opening, the size of the openings around the thighs, and the length or "pitch" of the diaper.

Articles worn externally to the body of the wearer, such as diapers, are commonly misapplied. This is due to various causes, including awkward positioning of the wearer or the restless movement of the wearer during fitting. Such misapplication may result in an uneven fit; gaps, which result in leakage; and misplaced parts (such as fasteners), which may result in marking the skin of the wearer and/or discomfort. This is particularly likely to occur with caregivers dealing with uncooperative wearers such as babies. Babies, even from a young age, move their legs into awkward positions, roll from side to side, or even violently resist diaper changes using hand and leg motions. As a result, the caregiver often has to hold portions of the wearer's body as well as the diaper during the change process, making it very difficult to achieve proper alignment of the diaper for fitting.

Attempts have been made in the art to improve the process of applying an absorbent article to the wearer such as by using an external change aid, such as described in European Patent Application No. 01117671, filed July 26, 2001. The change aids described in that application are devices that assist in the application or removal of articles worn primarily externally on the body of the wearer, especially hygienic absorbent articles, such as diapers, adult incontinence articles, feminine protection articles, and the like. However, such devices are not integrated in the absorbent article itself, requiring the caregiver to purchase an extra device.

Thus, there is a need for an absorbent article, such as a disposable diaper, that includes features that facilitate the changing process. Particularly, there is a need for a diaper having intuitive elements that facilitate a change by enabling a caregiver to fit the absorbent article on the wearer correctly the first time with minimal, if any, adjustment. There is also a need for a diaper having versatile change elements that enable it to be fitted to a wearer while the wearer is standing or lying down, where in either case the diaper can easily be fitted to the wearer without instruction.

### SUMMARY OF THE INVENTION

A disposable absorbent article having curved serviceable indicia to facilitate fitting of the disposable absorbent article on the wearer is provided. The disposable absorbent article is configured to be worn about the lower torso of a wearer. The serviceable indicium may aid in aligning the disposable absorbent article to the wearer or may provide guidance on how to grasp the disposable absorbent article during fitting of the disposable absorbent article to the wearer. The disposable absorbent article includes a body-facing surface and a garment-facing surface; a longitudinal axis and a transverse axis; a front waist region with a first end edge, a rear waist region with a second end edge, and a crotch region interposed therebetween. A pair of opposing longitudinal side edges join the first end edge and the second end edge. The disposable absorbent article includes a backsheet having a body-facing surface and a garment-facing surface, and a core disposed on the body-facing surface of the backsheet.

In one embodiment, the disposable absorbent article includes at least one first externally visible serviceable indicium proximate a front waist region of the disposable absorbent article. The first serviceable indicium has a curvature for alignment with an anatomical feature navel of the wearer.

In a further embodiment, the disposable absorbent article comprises preferably first an second barrier leg cuffs and first and second front ear panels for example formed in part by the first and second further barrier leg cuffs. The absorbent article further includes further serviceable indicia (160) the first and second front ear panels. The first and second serviceable indicia may indicate to a caregiver where to grasp the first and second front ear panels.

Additional aspects of the disclosure are defined by the claims of this patent. While multiple embodiments are disclosed herein, still other embodiments of the invention will become apparent to those skilled in the art from the following detailed description, which shows and describes illustrative embodiments of the invention. As will be realized, the invention is capable of modifications in various obvious aspects, all without departing from the spirit and scope of the invention. Accordingly, the drawings and detailed description are to be regarded as illustrative in nature and not restrictive.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings, in which like designations are used to designate substantially identical elements, and in which:
Figure 1A is a plan view of a disposable absorbent article suitable for use with the present invention.
Figure 1B is a plan view of an alternative embodiment of a disposable absorbent article suitable for use with the present invention.
Figure 2 is a plan view of a portion of a disposable absorbent article having a first curved serviceable indicium proximate a front waist region in accordance with one embodiment.
Figure 3 is a plan view of a portion of a disposable absorbent article having a first curved serviceable indicium proximate a front waist region and a landing zone in accordance with one embodiment.
Figure 4 is a plan view of a portion of a disposable absorbent article having a first curved serviceable indicium proximate a front waist region and a landing zone having a complementary curved serviceable indicium in accordance with one embodiment.
Figure 5 is a plan view of a portion of a disposable absorbent article having a first curved serviceable indicium proximate a front waist region and a landing zone forming a second curved serviceable indicium in accordance with one embodiment.
Figure 6 is a plan view of a portion of a disposable absorbent article having a first curved serviceable indicium proximate a first waist region, a landing zone, front ear panels, and a further serviceable indicium provided between a front ear panel and the landing zone in accordance with one embodiment.
Figure 7 is a plan view of a portion of a disposable absorbent article having a first curved serviceable indicium proximate a first waist region, a landing zone forming a second curved serviceable indicium, and third and fourth serviceable indicium along the lateral edges of the front waist region in accordance with one embodiment.
Figure 8A is a plan view of a portion of a disposable absorbent article having a first curved serviceable indicium proximate a first waist region, a landing zone forming a second curved serviceable indicium, and a third serviceable indicium provided around the landing zone in accordance with one embodiment.
Figure 8B is a plan view of a portion of a disposable absorbent article having a first curved serviceable indicium, second curved serviceable indicium, and third curved serviceable indicium merged together.
Figure 9 is a plan view of a portion of a disposable absorbent article having a first curved serviceable indicium proximate a front waist region and a second curved serviceable indicium longitudinally spaced from the first curved serviceable indicium in accordance with one embodiment.
Figure 10 is a plan view of a portion of a disposable absorbent article having front ear panels and a further serviceable indicium provided along at least one of the front ear panels in accordance with one embodiment.
Figure 11 is a plan view of a portion of a disposable absorbent article having front ear panels and a further serviceable indicium provided along at least one of the front ear panels, the serviceable indicium including a graphic, in accordance with one embodiment.
Figure 12 is a plan view of a portion of a disposable absorbent article having front ear panels, further serviceable indicia provided along the front ear panels, and a first serviceable indicium provided proximate a front waist region in accordance with one embodiment.
Figure 13 is a plan view of a portion of a disposable absorbent article having front ear with further panels, with further serviceable indicia provided along the front ear panels, a first serviceable indicium provided proximate a first waist region, and a curved landing zone forming second a second serviceable indicium in accordance with one embodiment.
Figure 14 is a plan view of a portion of a disposable absorbent article having front ear panels, further serviceable indicia provided along the front ear panels, a first serviceable indicium provided proximate a first waist region, and a landing zone having no curvature in accordance with one embodiment.
Figure 15 is a plan view of a portion of a disposable absorbent article having front ear further serviceable indicia provided along the front ear panels, a first serviceable indicium provided proximate a first waist region, and second serviceable indicium first provided longitudinally spaced from the first serviceable indicium in accordance with one embodiment.
Figure 16 is a plan view of the body-facing surface of the disposable absorbent article according to the present invention, including internally visible serviceable indicia.
Figure 17 is an isometric view of the body-facing surface of the disposable absorbent article according to the present invention, including an internally visible serviceable indicium, which may be both colored and 3-dimensional.
Figure 18 shows the disposable absorbent article depicted in Figure 17 being fitted to a wearer.
Figure 19 is a plan view of a diaper including instructional serviceable indicia on the landing zone denoting a first fit and a second fit.

### DETAILED DESCRIPTION OF THE INVENTION

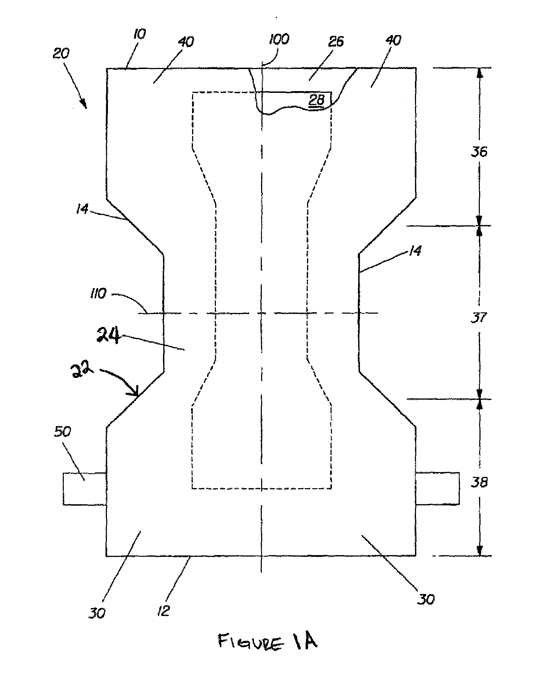

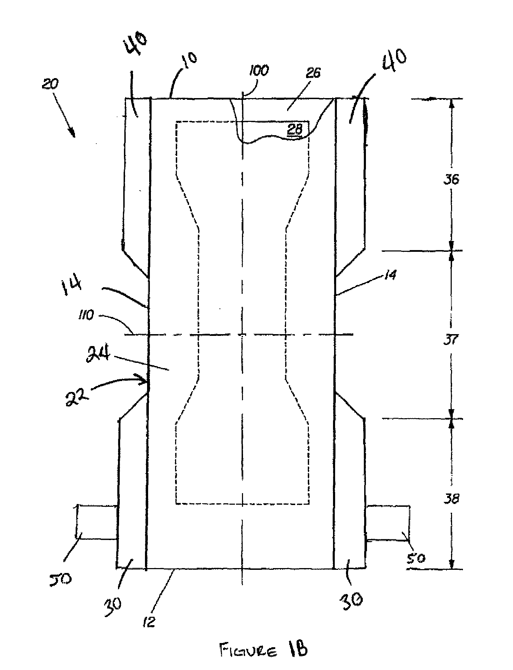

A disposable absorbent article including at least one serviceable indicium that facilitates an easy intuitive change is provided. The disposable absorbent article is configured for wearing about the lower torso of a wearer. The serviceable indicium is disposed in distinct areas of the article and includes features and/or characteristics signaling to a caregiver and/or wearer how to achieve a proper fit. The wearable article may be applicable to disposable absorbent articles including training pants, incontinence briefs, incontinence undergarments, inserts for disposable or durable diapers or other garments, and the like. One embodiment of an absorbent article comprises a unitary disposable absorbent article, such as the disposable diaper 20 shown in Figure 1.

A disposable absorbent article to be worn about the lower torso of a wearer that facilitates an easy, intuitive change is provided. The disposable absorbent article includes at least one serviceable indicium that facilitates fitting of the disposable absorbent article to the wearer. The serviceable indicium may aid in aligning the disposable absorbent article to the wearer, or may provide guidance on how to grasp the disposable absorbent article during fitting of the disposable absorbent article to the wearer.

### DEFINITIONS

As used herein, the following terms have the following meanings:
"Absorbent article" refers to devices that absorb and contain liquid, and, more specifically, refers to devices that are placed against or in proximity to the body of the wearer to absorb and contain the various exudates discharged from the body.
"Longitudinal" is a direction running parallel to the maximum linear dimension of the article and includes directions within ±45° of the longitudinal direction.
The "lateral" or "transverse" direction is orthogonal to the longitudinal direction and is essentially in the plane of the article when the article is in a flat stretched out position.
The "Z-direction" is orthogonal to both the longitudinal and transverse directions.
The "x-y plane" refers to the plane congruent with the longitudinal and transverse directions.
The term "disposable" is used herein to describe absorbent articles that generally are not intended to be laundered or otherwise restored or reused as an absorbent article (i.e., they are intended to be discarded after a single use and, preferably, to be recycled, composted, or otherwise disposed of in an environmentally compatible manner).
The term "disposed" is used herein to mean that an element(s) is formed (joined and positioned) in a particular place or position as a unitary structure with other elements or as a separate element joined to another element.

As used herein, the term "joined" encompasses configurations whereby an element is directly secured or coupled to another element by affixing the element directly to the other element, and configurations whereby an element is indirectly secured or coupled to another element by affixing the element to intermediate member(s) that in turn are affixed to the other element.

A "unitary" absorbent article refers to an absorbent article formed of separate parts united together to form a coordinated entity, such that it does not require separate manipulative parts like a separate holder and liner.

As used herein, the term "diaper" refers to an absorbent article generally worn by infants, toddlers, and incontinent persons about the lower torso.

As used herein, the term "complement" refers to filling in or completing, such as by overlapping, matching, or aligning therewith, contextually relating, or highlighting.

As used herein, the term "body-facing surface" generally refers to a surface oriented toward the body when fitted to a wearer.

As used herein, the term "garment-facing surface" generally refers to a surface oriented opposite the body-facing surface when fitted to a wearer.

As used herein, the term "serviceable indicium or indicia" generally refers to distinctive marks, colored regions, patterns, and/or textures disposed on a disposable absorbent article to provide a functional attribute. Specifically, the functional attribute includes providing a visual indication that facilitates an easy intuitive change of the disposable absorbent article during fitting. The serviceable indicium may include multiple indicia that define a curve, region, or continuous or discrete elements. The serviceable indicium may be illustrated via shapes, and/or colors, and/or graphics, and/or instructional material, and/or character faces, and/or dots, and/or numbers, and/or line segments, and/or patterns, etc. There may be serviceable indicia within other serviceable indicia.

The term "externally visible," as used in reference to an indicium associated with an article, refers to indicium that may be visually discerned with the unaided eye (excepting standard corrective lenses adapted to compensate for near-sightedness, farsightedness, or astigmatism) in standard lighting conditions from a point of reference viewing the garment-facing surface of the article while the article is held in a configuration wherein the garment-facing surface is within the field of view.

As used herein, the term "internally visible," as used in reference to an indicium associated with an article, refers to indicium that may be visually discerned with the unaided eye (excepting standard corrective lenses adapted to compensate for near-sightedness, farsightedness, or astigmatism) in standard lighting conditions from a point of reference viewing the body-facing surface of the article while the article is held in a configuration wherein the body-facing surface is within the field of view.

As used herein, "standard lighting conditions" refer to lighting conditions in which human vision operates efficiently (e.g., the human eye is able to discern complex patterns, shading, and colors). Specifically, standard lighting conditions are at least one of the following: natural illumination as experienced outdoors during daylight hours; the illumination of a standard 100 watt incandescent white light bulb at a distance of 2 meters; or as defined by CIE D65 standard illuminate lighting at 800 lux to a 1964 CIE standard observer.

As used herein, an "anatomic feature" of a wearer may include any externally discernible portion of the wearer's anatomy specific to a certain definable region and/or function. Exemplary anatomic features of human bodies include, without limitation, waste exit ports such as the anus, genitalia, the perineal region, the gluteal groove, leg creases, the navel, buttocks, hip and/or pubic bones, the thighs, the rib cage, and the like.

Figure 1A is a plan view of a disposable absorbent article 20 suitable for use with the present invention in its flat, uncontracted state (i.e., without elastic induced contraction), with portions of the structure being cut away to more clearly show the underlying structure of the disposable absorbent article 20 and with the body-facing portion of the diaper 20 that contacts the wearer facing the viewer. As shown, the disposable absorbent article 20 comprises a diaper. The diaper 20 includes a longitudinal axis 100 and a lateral or transverse axis 110, and a first end edge 10 and a second end edge 12 connected by longitudinally extending side edges. A longitudinal side edge refers to an edge oriented ±45° from the longitudinal axis 100 and includes rectilinear and curvilinear side edges. One end portion of the diaper 20 is configured as a front waist region 36 of the diaper 20. The opposite end portion is configured as a rear waist region 38 of the diaper 20. An intermediate portion of the diaper 20 is configured as a crotch region 37, which extends longitudinally between the first and second waist regions 36 and 38. The waist regions 36 and 38 generally comprise those portions of the diaper 20 that, when worn, encircle the waist of the wearer. Therefore, the front waist region 36 and the rear waist region 38 are commonly referred to as the front waist region and the back waist region, respectively, to correspond to orientation of the diaper 20 relative to the wearer's body during fit. The waist regions 36 and 38 may include elastic elements that gather about the waist of the wearer to provide improved fit and containment. The elastic elements may extend over the entire width of the waist regions 36, 38, along only a portion of the waist regions 36, 38, or may not be provided at all. The crotch region 37 is that portion of the diaper 20 that, when the diaper 20 is worn, is generally positioned between the legs of the wearer.

A chassis 22 of the diaper 20 comprises the main body of the diaper 20. The chassis 22 comprises an outer covering and includes a liquid pervious topsheet 24 and/or a liquid impervious backsheet 26 and at least a portion of an absorbent core 28 encased between the topsheet 24 and the backsheet 26. For unitary absorbent articles, the chassis 22 comprises the main structure of the diaper 20, with other features added to form the composite diaper structure. While the topsheet 24, the backsheet 26, and the absorbent core 28 may be assembled in a variety of well-known configurations, exemplary diaper configurations are described generally in U.S. Patent Nos. 3,860,003, 5,151,092, 5,221,274, 5,554,145, 5,569,234, 5,580,411, and 6,004,306,.

The topsheet 24 may be fully or partially elasticized or may be foreshortened so as to provide a void space between the topsheet 24 and the core 28. Exemplary structures including elasticized or foreshortened topsheets are described in more detail in U.S. Patent Nos. 4,892,536, 4,990,147, 5,037,416, and 5,269,775, each of which is incorporated herein by reference.

The absorbent core 28 may comprise any absorbent material that is generally compressible, conformable, non-irritating to the wearer's skin, and capable of absorbing and retaining liquids such as urine and other certain body exudates. The absorbent core 28 may comprise a wide variety of liquid-absorbent materials commonly used in disposable diapers and other absorbent articles such as comminuted wood pulp, which is generally referred to as air felt. Examples of other suitable absorbent materials include creped cellulose wadding; melt blown polymers, including co-form; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The backsheet 26 is generally that portion of the diaper 20 positioned adjacent the garment-facing surface of the absorbent core 28. The backsheet 26 prevents the exudates absorbed and contained therein from soiling articles that may contact the diaper 20, such as bed sheets and garments. In one embodiment, the backsheet 26 is substantially impervious to liquids (e.g., urine) and comprises a laminate of a nonwoven and a thin plastic film such as a thermoplastic film having any suitable thickness, such as about 0.012 mm (0.5 mil) to about 0.051 mm (2.0 mils). Suitable backsheet films include those manufactured by Tredegar Industries Inc. of Terre Haute, IN and sold under the trade names X15306, X10962, and X10964. Other suitable backsheet materials may include breathable materials that permit vapors to escape from the diaper 20 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co. of Japan under the designation ESPOIR NO and by Tredegar, of Richmond, VA. Suitable breathable composite materials comprising polymer blends are available from Clopay Corporation, Cincinnati, OH, under the name HYTREL blend P18-3097. Any suitable materials may be used to construct the articles of the present invention.

The diaper 20 may include a fastening system 50. Any suitable fastening system may be used. For example, a hook-and-loop-type fastener including at least one engaging component (male fastening component) and at least one landing zone (female fastening component) may be used. In this embodiment, the fastening system 50 includes the fastener and the landing zone with the landing zone being provided on a central portion of the exterior of the diaper 20. Alternatively, the fastening system 50 may include a tab-and-slot-type fastener wherein the tab member includes a retaining element that interlocks with an opening such as a slit, slot, or loop.

The diaper 20 may also include side panels, referred to herein as rear ear panels 30, disposed in the rear waist region 38. The rear ear panels 30 may be integral with the chassis 22, comprising extensions of at least one of the materials of the chassis 22, e.g. topsheet 24, backsheet 26, as shown in Figure 1A, or, alternatively, the rear ear panels 30 may comprise separate members attached to the chassis 22 using adhesives, ultrasonic bonds, radio frequency bonds, or other suitable means, as shown in Figure 1B. The rear ear panels 30 may be elastic or extensible to provide a comfortable and contoured fit by initially conformably fitting the diaper 20 to the wearer and sustaining this fit throughout the time of wear well past when the diaper 20 has been loaded with exudates, since the elasticized rear ear panels 30 allow the sides of the diaper 20 to expand and contract. The rear ear panels 30 may also provide more effective application of the diaper 20 because, even if the caregiver pulls one elasticized first ear panel 30 farther than the other during application, the diaper 20 will "self-adjust" during wear. Examples of diapers with elasticized rear ear panels are disclosed in U.S. Patent Nos. 4,857,067, 4,381,781, 4,938,753, 5,151,092, 5,221,274, and 5,669,897, and PCT WO 95/13775,.

The diaper 20 also can include side panels, referred to as front ear panels 40, disposed in the front waist region 36. Similar to the rear ear panels 30, the front ear panels 40 may be integral with the chassis 22, comprising extensions of a unibody chassis design, as shown in Figure 1A, or, alternatively, the front ear panels 40 may comprise separate members attached to the chassis 22 using adhesives, ultrasonic bonds, radio frequency bonds, or other suitable means, as shown in Figure 1B. The rear and front ear panels 30, 40 may be constructed in any suitable configuration accommodating a particular product design.

Generally, the rear ear panels 30 are provided for fixing the rear waist region 38 to the front waist region 36. The front ear panels 40 are generally used as handling means for aligning and placing the diaper on the wearer. Thus, in fitting the diaper 20 to the wearer, the caregiver pulls the rear ear panels 30 forwardly around the sides of the wearer and the front ear panels 40 rearwardly around the sides of the wearer, with the rear ear panels 30 overlaying the front ear panels. Where the fastening system is hook-and-loop-type fastener and the rear ear panels form the engaging component, the rear ear panels are then adjusted and placed on the landing zone.

In certain embodiments, the disposable absorbent article 20 can include at least one serviceable indicium providing guidance or instruction to the caregiver relative to the proper fit of the diaper or the manner of fitting the diaper to the wearer. In particular, in one embodiment, the serviceable indicium may provide an alignment indicator for aligning the diaper with an anatomical feature of the wearer. In another embodiment, the serviceable indicium may indicate to the caregiver where to grasp portions of the diaper to fit the diaper to the wearer. The serviceable indicium may include dots, graphics of characters, numbers, line segments, and/or patterns.

The disposable diaper 20 includes at least one externally visible serviceable indicium that is observable along at least portions of the garment-facing surface of the article proximate the front waist region 36 or proximate the longitudinal side edges or front ear panels 40. In each portion, the serviceable indicia are externally visible so as to distinguish portions of the garment facing surface of the article proximate the front waist region 36 or front ear panels 40 from other portions of the diaper 20. The externally visible serviceable indicia may be disposed directly on the garment-facing surface of the backsheet 26, on the surface of the backsheet 26 opposite the garment-facing surface adjacent to the core 28, on one of the components of the backsheet 26, or beneath the backsheet 26 on underlying layers so long as the indicia are externally visible. The externally visible serviceable indicia may comprise a separate element affixed to a component of the article, or a colorant, such as a dye or ink, applied to a component of the article. In addition, the externally visible serviceable indicia may include a color, a pattern, and/or a texture that distinguish the designated portions from the center portion of the article. Any suitable indicial may be used.

For externally visible serviceable indicia comprising a pattern, the pattern may be in the form of a series of shapes and/or images. For example, the pattern may be formed of one or more dots, one or more lines, one or more regular or irregular shapes (such as circles, ellipses, diamonds, squares, and the like), or combinations thereof. Alternatively, or in conjunction with the shapes and/or images, a pattern may be in the form of a variation of color along a length of the serviceable indicia. For example, the color may vary from light to dark or from one hue to another. Images may include drawings of characters or objects readily recognizable to children.

For serviceable indicia comprising texture, portions of the backsheet 26 may be mechanically treated to provide texture. Any suitable method may be used to provide texture, including operations such as pleating, corrugating, or ring rolling to provide folds that are able to open when the backsheet 26 is extended in a direction generally orthogonal to the pleats or folds. In addition to providing texture, these operations also may provide extensibility. Suitable processes for ring rolling or pre-corrugating, including extensible webs made thereby, are described in U.S. Patent Nos. 4,107,364, 4,834,741, 5,167,897, and 5,702,382, each of which is hereby incorporated herein by reference.

In some embodiments, serviceable indicia comprising texture may be accomplished by forming a strainable network having at least two contiguous, distinct, and dissimilar regions. Films thus formed have in the past been termed structural elastic-like films ("SELF"). A structural elastic-like film or web is an extensible material that can exhibit an elastic-like behavior in the direction of elongation without the use of added elastic materials. However, SELF webs can be made which exhibit little elastic behavior. In particular, webs comprising a laminate of films and nonwovens can be made which exhibit little elastic behavior beyond very low levels of strain.

Serviceable indicia comprising SELF suitable for the present invention, and methods of forming SELF webs suitable for use as backsheets 26, are more completely described in U.S. Patent Nos. 5,518,801, 5,650,214, and 5,904,673, all of which are hereby incorporated herein by reference. For film/nonwoven laminate backsheets, the processes described in the above-mentioned patents can be performed on the laminate material, or on the separate components prior to lamination, or both.

In certain embodiments, the serviceable indicium may be sufficiently opaque, or have a sufficiently dark color, to additionally provide a masking benefit, effectively preventing visual detection of a layer, material, or substance underlying the serviceable indicia. For example, where suitable, the serviceable indicium may have an opacity such that feces and/or portions of the absorbent core that may be present under the region of the backsheet are not visible from outside the diaper 20.

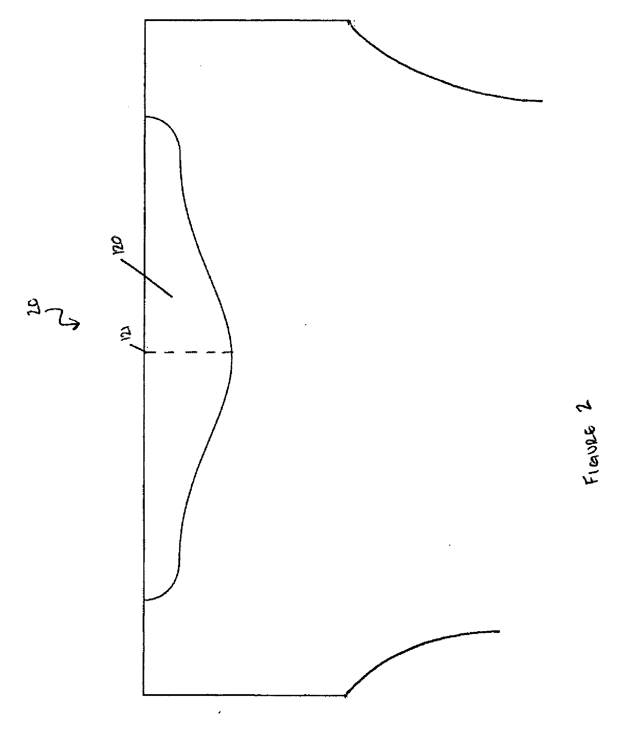

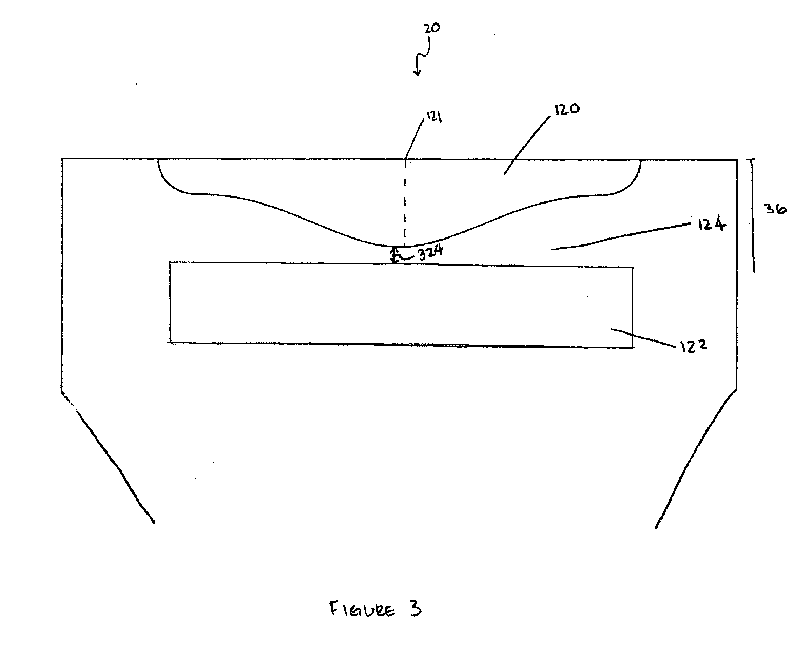

As shown in Figures 2 through 9, in accordance with various embodiments a first serviceable indicium 120 is provided proximate the front waist region 36 for alignment with an anatomical feature of the wearer, such as the navel of the wearer. The serviceable indicia may be any indicia contrasting with a portion of central region (crotch region 37) and/or a portion of an immediately adjacent region of the diaper that allows a caregiver to fit the diaper properly on the wearer. For example, as shown in Figures 2 through 9, the first serviceable indicium 120 comprises curvature which can be aligned with an anatomic feature of the wearer thereby assisting the caregiver in properly aligning the article on the wearer. Referring to Figures 3 through 9, the landing zone 122 of the fastening system 50, if provided, may also have a curvature or be aligned with a curvature. As shown, in some embodiments, the curvature of the first serviceable indicium 120 may be complementary with the curvature of the landing zone 122. In some embodiments, the first serviceable indicia 120 and/or the curved landing zone 122 can impart the appearance of underwear to the diaper 20, making the diaper more appealing to toddlers. Further, the configuration of the first serviceable indicium 120 can be convex relative to the lateral centerline of the diaper. Such convex pattern, particularly the pattern proximate the front end edge, can complement the shape of the wearer's belly. Any desired serviceable indicium 120 may be used.

As shown in Figure 2, an externally visible first serviceable indicium 120 is provided within the front waist region 36 of the diaper 20. Generally, the first serviceable indicium 120 can be provided proximate the front waist edge. As shown, the first serviceable indicium 120 can be convex with respect to the lateral centerline of the disposable absorbent article 20. Thus, a minima point 121 is provided in the lateral direction that can be approximated with the wearer's navel, thereby enabling a caregiver to easily and properly align the diaper for fitting.

Figure 3 illustrates the embodiment of Figure 2 further including a fastening system including a landing zone 122. As discussed previously, a landing zone is commonly provided with hook-and-loop-type fasteners. The fastening system includes at least one engaging component (male fastening component), generally extending from the rear waist region 38, and at least one landing zone 122 (female fastening component). The landing zone 122 can be longitudinally spaced from the first serviceable indicium 120, thereby forming a gap 124. The gap 124 provides an enhanced image to the caregiver of where to place the fasteners of the fastening system to help the caregiver avoid confusing the landing zone 122 with the indicia.

The gap 124 may have a gap width 324. The gap width 324 is the smallest straight line distance between the two closest points of the first serviceable indicium 120 and the landing zone 122. The gap width 324 may be of any suitable size. For example, the gap width 324 can be between about 0.5 mm to about 40 mm, or any individual number or any range within this range. As another example, the gap width 324 can be between about 2 mm to about 20 mm.

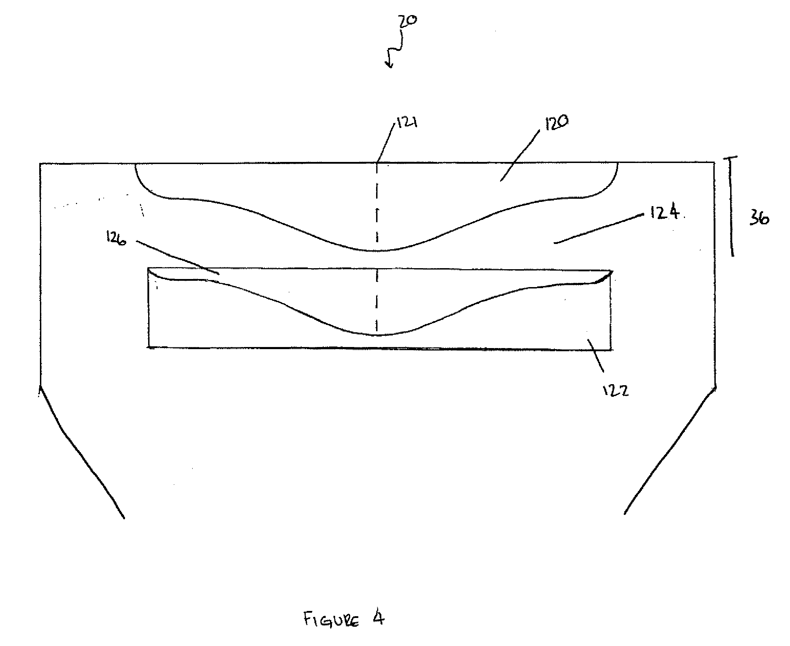

The landing zone 122 may further include a graphic or other forming a second serviceable indicium, or may itself form a second serviceable indicium. Thus, Figure 4 illustrates an embodiment wherein a graphic serviceable indicium 126 is provided on the landing zone 122. As shown, the graphic serviceable indicia 126 comprises a graphic curvature generally parallel to the curvature of the first serviceable indicium 120. Other graphics alternatively may be used. Additionally, in some embodiments, the graphic serviceable indicium 126 may be complementary to the first serviceable indicium 120.

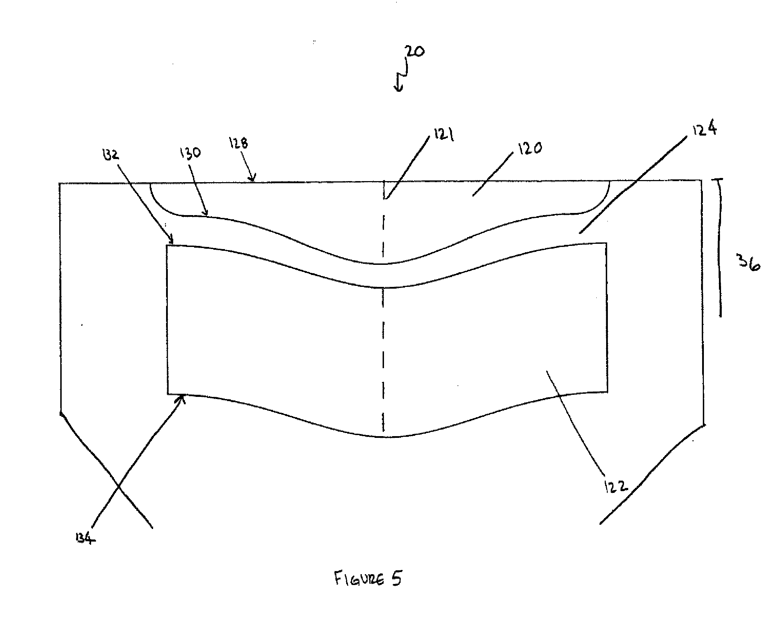

As shown in Figure 5, in some embodiments, the landing zone 122 has a complementary curvature to the serviceable indicium 120 provided proximate to the front waist region 36. The landing zone 122 thus forms a second indicium. A gap 124 is provided between the first serviceable indicium 120 and the landing zone 122. Thus, in the embodiment of Figure 5, the first serviceable indicium 120 has a first edge 128 proximate the front waist edge and a second edge 130 longitudinally inward of the first edge 128. The landing zone 122 has a first edge 132 generally proximate the second edge 130 of the first serviceable indicium 120 and a second edge 134 longitudinally inward of the first edge 132. The second edge 130 of the first serviceable indicium 120 can be generally parallel to the first edge 132 of the landing zone 122 over at least a portion of their lengths. For example, the second edge 130 of the first serviceable indicium 120 may be generally parallel to the first edge 132 of the landing zone 122 for at least about two centimeters, or for any suitable distance. Thus, the gap 124 remains approximately constant over at least a portion of the length of the landing zone 122 and the serviceable indicium 120. For example, the gap 124 may remain approximately constant over any desired length, including about 50% of the length, of the landing zone 122 and the serviceable indicium 120. In various embodiments, the landing zone 122 may be convex relative to the lateral axis of the article, may be concave relative to the lateral axis of the article, or may be parallel to the lateral axis of the article. In some embodiments, the first edge 132 of the landing zone 122 can be generally convex with respect to the lateral axis while the second edge 134 of the landing zone 122 can be generally parallel to the lateral axis of the article. In some embodiments, both the first edge 132 and the second edge 134 of the landing zone 122 can be generally parallel to each other over any desired length.

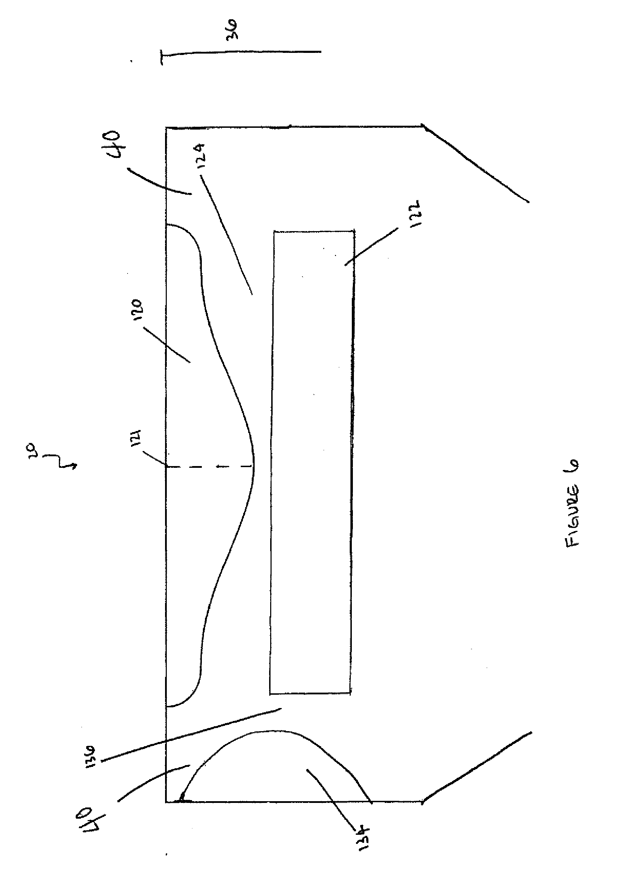

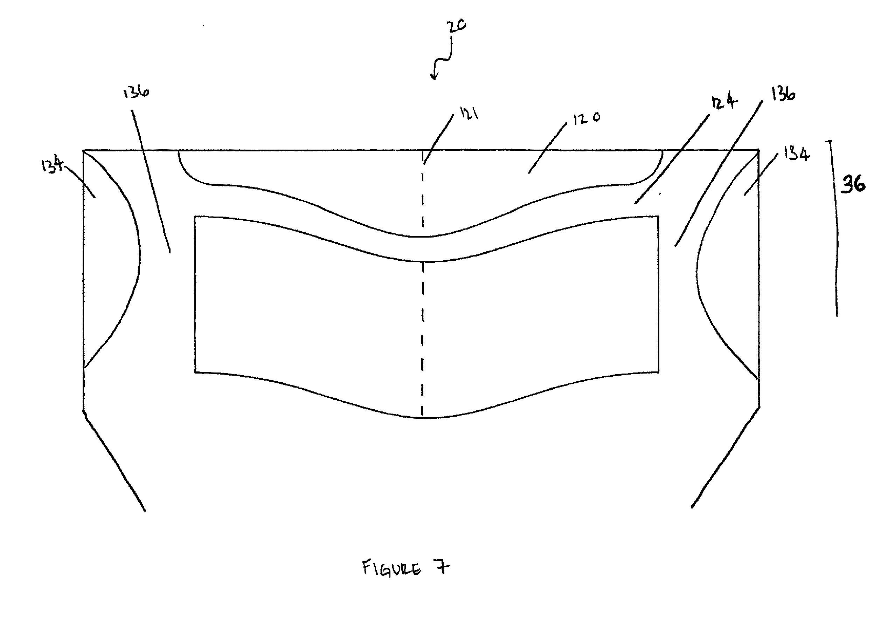

In some embodiments, further serviceable indicia may be provided laterally outwardly from the landing zone 122. Thus, Figure 6 illustrates an alternative embodiment of the disposable absorbent article 20 of Figure 3 and Figure 7 illustrates an alternative embodiment of the disposable absorbent article 20 of Figure 5.

In the embodiment of Figure 6, front ear panels 40 are provided extending laterally outward from the front waist region 36. The front ear panels 40 may be integral with the chassis 22 comprising extensions of at least one of the materials of the chassis, or may comprise separate members attached to the chassis using adhesives, ultrasonic bonds, radio frequency bonds, or any other suitable means. Generally, the front ear panels 40 are grasped by a caregiver during fitting of the diaper to maneuver the front of the diaper into the correct position on the wearer's torso. A third serviceable indicium 134 is provided proximate a position of one of the front ear panels 40. A further serviceable indicium may be provided proximate the other second ear panel 40. The third serviceable indicium 134 is spaced from the landing zone 122 to form a further gap 136.

Figure 7 illustrates a disposable absorbent article 20 having a first serviceable indicium 120 proximate the front waist region 36, a second serviceable indicium comprising a landing zone 122 having a curvature generally complementary to a curvature of the first serviceable indicium 120, and third serviceable indicia 134 can be laterally spaced from the landing zone 122 to form gaps 136. As discussed with respect to Figure 6, the third serviceable indicia 134 may be provided generally proximate to front ear panels.

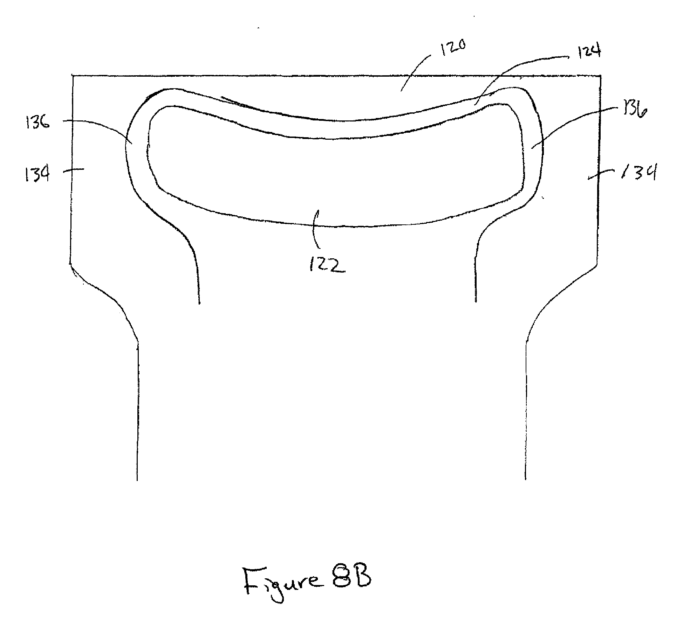

As shown in Figures 6 and 7, the gaps 124 and 136 may be generally discontinuous or segmented, which in itself provides suitable indicia. However, in some embodiments, the gaps 124 and 136 can be continuous, for example as shown in Figure 8B.

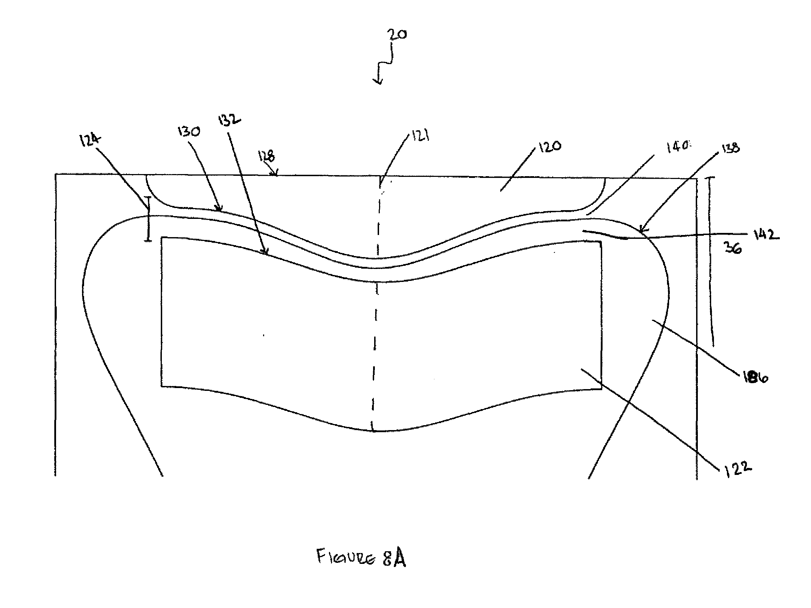

As shown in Figure 8A, in some embodiments, a first serviceable indicium 120 can be provided proximate the front waist region 36. A second serviceable indicium can be provided comprising a landing zone 122 having a curvature generally complementary to a curvature of the first serviceable indicium 120. In some embodiments, the second serviceable indicium may be provided on the landing zone 122, as shown in Figure 4. In some embodiments, the landing zone 122 may comprise curvature and may be complementary to the first serviceable indicia 120. In some embodiments, the landing zone 122 may not have a curvature, or no second serviceable indicium may be provided. As shown in Figure 8A, in some embodiments, a gap 124 can be provided between the first serviceable indicium 120 and the landing zone 122. A fourth serviceable indicium 186 can be provided longitudinally spaced from the first serviceable indicium 120 and generally supporting the landing zone 120. The fourth serviceable indicium 186 may comprise a graphic such as a printed graphic, may be unitary with the chassis, or may comprise a separate member attached to the chassis using adhesives, ultrasonic bonds, radio frequency bonds, or other suitable means. In some embodiments, the top edge 138 of the fourth serviceable indicium 186 can split the gap 124 into a first serviceable indicium gap 140 between the edge 130 of the first serviceable indicium 120 and the top edge 138 of the fourth serviceable indicium 186 and a landing zone gap 142 between the top edge 132 of the landing zone 122 and the top edge 138 of the fourth serviceable indicium 186. The fourth serviceable indicium 186 can be distinct from the first serviceable indicia 120 and/or the third serviceable indicia 134.

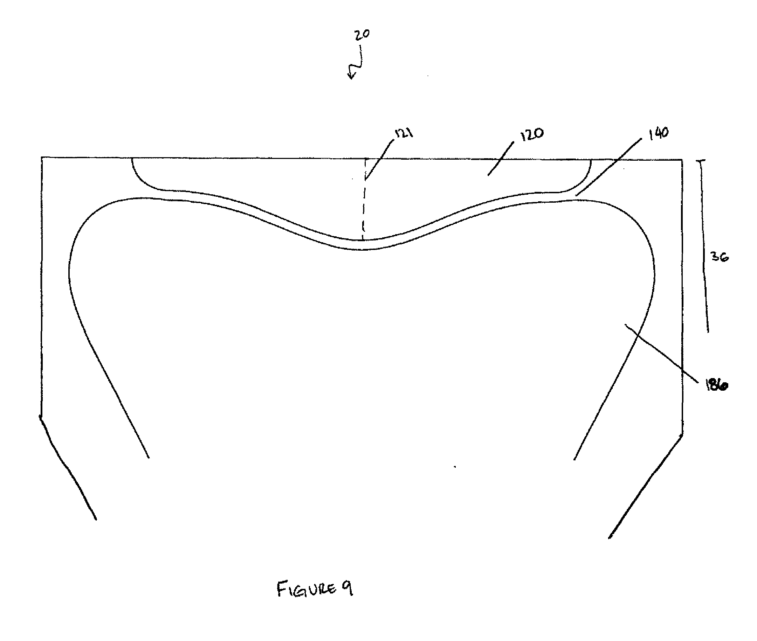

Figure 9 illustrates the embodiment of Figure 8 without a landing zone. This embodiment thus may be used with other types of closures, for example a tab-and-slot-type fastener.

As shown in Figure 8B, in some embodiments, the first serviceable indicium 120 and the third serviceable indicia 134 can be merged. As shown, the gaps 124 and 136 can similarly be merged to form a continuous gap around the landing zone 122.

Absorbent articles constructed in accordance with the present invention may comprise any suitable combination of serviceable indicia. For example, an absorbent article constructed in accordance with the present invention may comprise the first serviceable indicium 120, the second serviceable indicium, the third serviceable indicium 134, the fourth serviceable indicium 186, and/or the graphic serviceable indicium 126 as discussed heretofore.

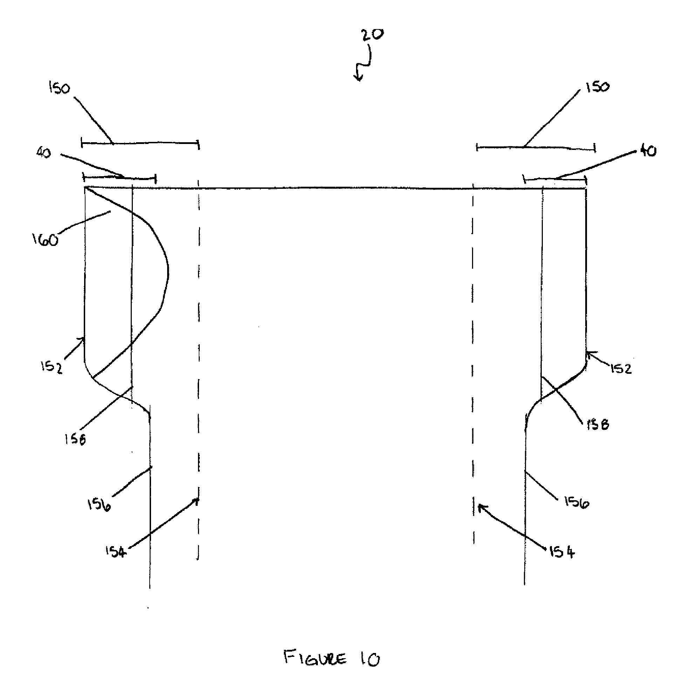

Figures 10-15 illustrate embodiments of a disposable absorbent article comprising a front ear panel forming at least a portion of the longitudinal edge of the article. The front ear panel may further be formed partially by a barrier cuff web of a barrier leg cuff. The barrier leg cuff is the portion of the diaper that, as fitted to the wearer, extends around the legs of the wearer. Additionally, the diaper may further include elastically contractible gasketing leg cuffs to provide better fit and capture of bodily exudates. Each of the gasketing leg cuffs is disposed outside a barrier leg cuff, adjacent to the longitudinal side edge. Thus, as shown in Figure 10, the disposable absorbent article 20 comprises first and second barrier leg cuffs 150 extending from first and second lateral edges 152 of the absorbent article 20 to an inward edge 154 of the barrier leg cuff material. Front ear panels 40 are formed between either the first and second lateral edge 152 and a lateral edge 156 of the chassis. As previously discussed, the diaper comprises a topsheet, a core, and a backsheet. In the embodiment of Figure 10, the backsheet material ends at a position 158 along the barrier leg cuff material. As shown, at least a portion of the front ear panels 40 may not comprise backsheet material and are formed at least in part by barrier leg cuff material by extending the barrier leg cuff material laterally outward. A further serviceable indicium 160 (can be configured similar to the third serviceable indicia 134) can be provided on at least one of the front ear panels 40. In some embodiments, the indicia may span the entire front ear 40. In some embodiments, the indicia may span from the lateral edge 152 further inward extending beyond the position 158. The serviceable indicium 160 serves to provide guidance on where to grasp the front ear panel 40.

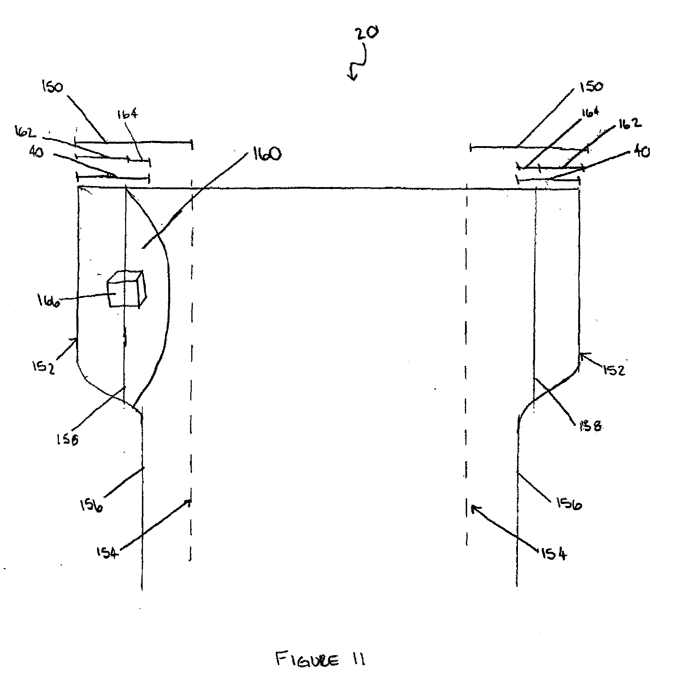

The embodiment of Figure 11 is a variation of the embodiment of Figure 10. As shown, the front ear panels 40 may comprise a laterally outward region 162 and a laterally inward region 164. The further serviceable indicium 160 may extend over the full expanse of the laterally outward region 162. As shown, the further serviceable indicium 160 may further comprise at least one graphic 166 to further aid in indicating where to grasp the front ear panel. The graphic 166 may be printed, dyed, provided as a separate piece of material adhered to the article 20, or otherwise provided.

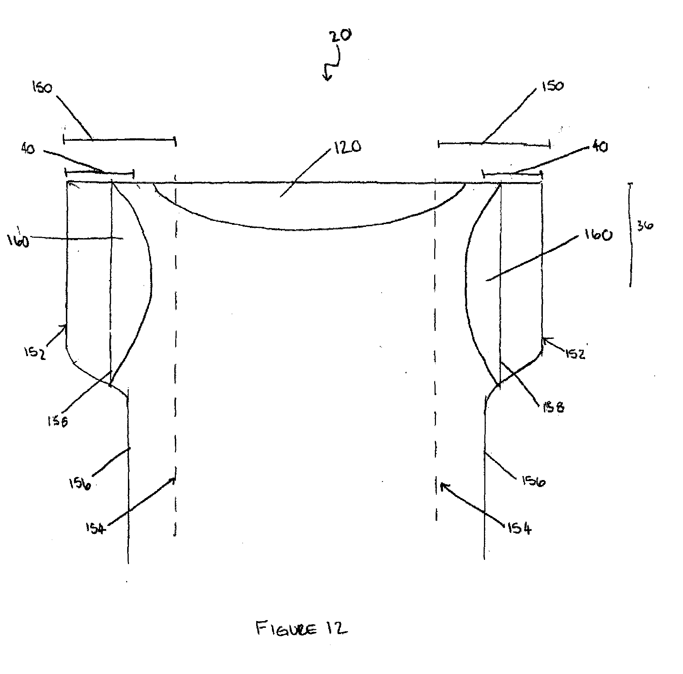

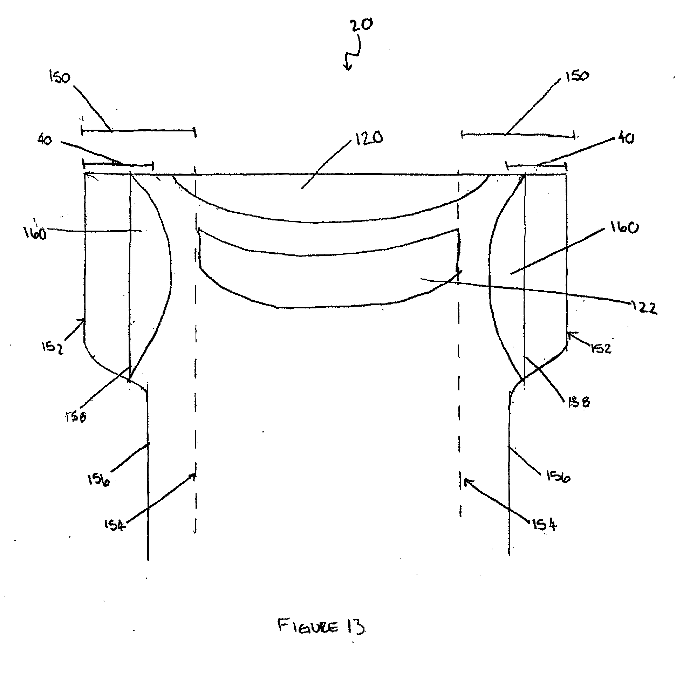

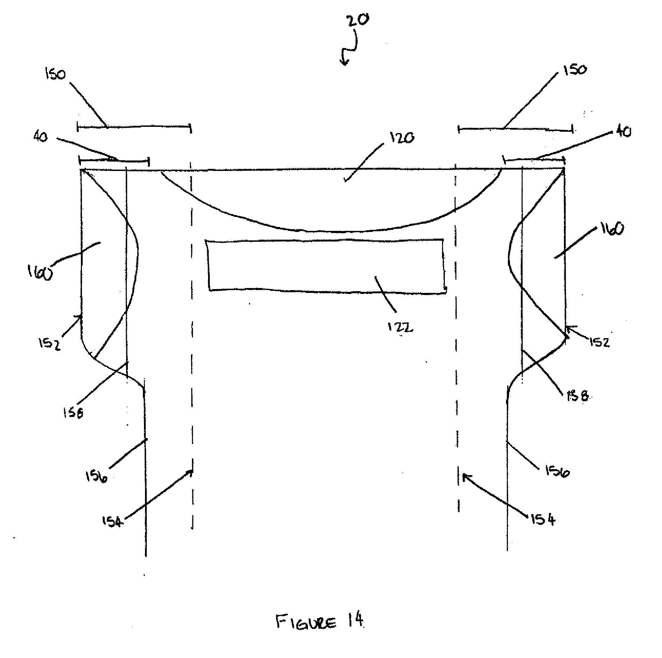

Figure 12 illustrates an embodiment wherein the disposable absorbent article 20 comprise further serviceable indicia 160 provided on first and second front ear panels 40, and further comprises the first serviceable indicium 120 generally proximate the front waist region 36. The first serviceable indicium 120 may comprise any of the variations described with reference to Figures 2-9. In some embodiments, as shown in Figure 13, the disposable absorbent article 20 may further comprise a landing zone 122. The landing zone 122 may have a curvature generally complementary to the curvature of the first serviceable indicium 120, as shown in Figure 13, or may have no curvature, as shown in Figure 14. The landing zone 122 may be configured as described heretofore. Additionally, the embodiment shown in Figure 13 may comprise the serviceable indicia described with respect to Figure 12.

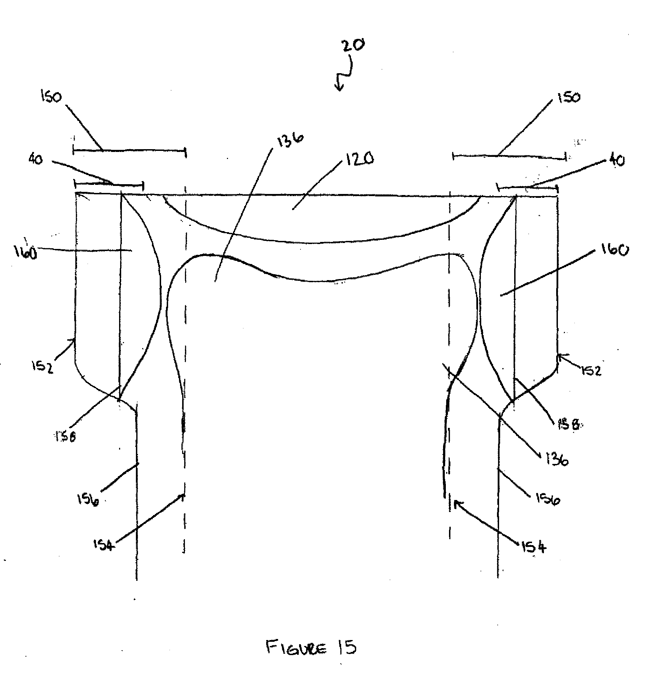

Figure 15 illustrates an embodiment wherein the disposable absorbent article 20 comprises further serviceable indicia 160 provided on first and second front ear panels 40, a third serviceable indicium 120 generally proximate the front waist region 36, and a fourth serviceable indicium 136 longitudinally spaced from the third serviceable indicium.

Figures 2 through 15 thus illustrate various embodiments of serviceable indicia suitable for use on a disposable absorbent article for aiding in fitting of the diaper to the wearer. In addition to aiding the fit of the diaper 20, a contoured pattern of serviceable indicia can be made to accentuate other features of the disposable absorbent article such as the absorbent core, the gasketing leg cuffs, or the barrier leg cuffs. For instance, the absorbent core 28 may have an hourglass shape design having a narrow portion in the crotch region 37 to improve the overall fit of the diaper 20. Additionally, the core 28 may comprise one or more rounded or otherwise shaped ends. The contoured shape formed by one or more serviceable indicia may complement or highlight the actual core shape or an idealized core shape (e.g., such as an idealized core shape having rounded ends on a diaper having a core with rectangular ends), making it more appealing to the consumer. In addition, the contoured pattern matching the core shape can provide a visual signal indicating proper alignment of the diaper 20 with respect to the wearer's lower torso, i.e., in the lateral direction, enhancing the fit of the diaper 20 in the wearer's crotch region 37, thus improving the overall performance of the diaper 20. On the other hand, the contoured pattern disposed on the garment-facing surface of the article may align with the barrier cuffs or gasketing leg cuffs disposed on the body-facing surface of the disposable absorbent article, accenting the articles ability to prevent leakage.

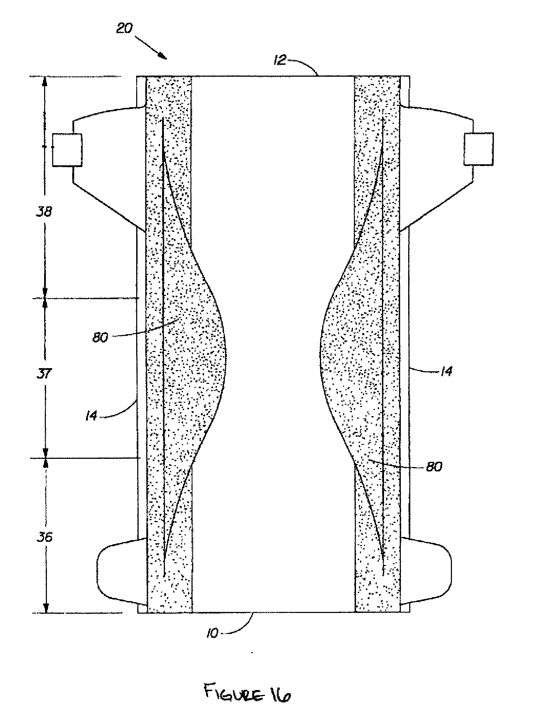

Referring to Figures 16-18, in some embodiments, the disposable absorbent article may comprise a body-facing surface including portions thereof having at least one internally visible serviceable indicium 80. The internally visible serviceable indicium 80 may facilitate an easy, intuitive change by providing a guide for aligning the wearer with the article during fitting so that in turn the wearer is accurately placed on the article for fastening, requiring minimal adjustment.

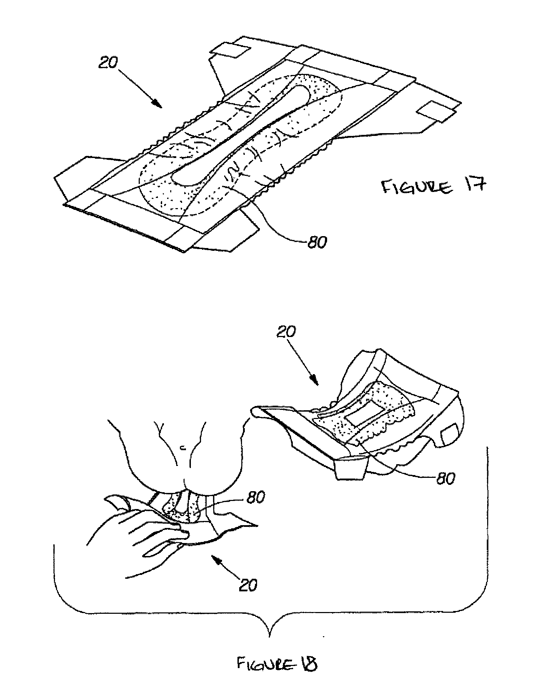

In certain non-limiting embodiments, internally visible serviceable indicia 80 may be disposed on the body-facing surface of the article to facilitate the placement or alignment of the article, or a component thereof, with respect to the lateral axis 110 and/or longitudinal axis 100 of the article with an anatomic feature of the wearer. For instance, the topsheet 24 of the article may comprise an internally visible serviceable indicium 80, such as a mark in the crotch region 37, that, when aligned such as to the anus, results in improved fit and waste containment performance of the article. In an embodiment shown in Figure 16, the internally visible serviceable indicium 80 is visible on the body-facing side of diaper 20 identifying the crotch area to aid in longitudinal positioning. In the embodiment shown in Figure 17, the internally visible serviceable indicium 80 may be colored, may include a three dimensional contour to facilitate alignment of the article with the wearer during fitting as shown in Figure 18, or both.

Similar to the embodiments including externally visible serviceable indicia such as shown and described with respect to Figures 2-15, the internally visible serviceable indicia 80 may include a color, a pattern, and/or a texture that distinguish the designated portions of the body-facing surface of the article from other regions of the body-facing surface not comprising the internally visible serviceable indicia 80. The internally visible serviceable indicia 80 may be associated with any portion or component of the article visible on or through the body-contacting surface of the article, including the topsheet 24, the absorbent core 28 or portions thereof, the fastening system 50, the topsheet 24, and the backsheet 26. In additional embodiments, the internally visible serviceable indicia 80 may be disposed proximate the longitudinal side edges 14 and proximate the first and second end edges 10, 12 of the article to provide a contoured pattern distinguishing the front waist region 36 from the rear waist region 38. The contoured pattern can be made to complement the wearer's anatomy, indicating to the caregiver where to place the wearer during fitting. For instance, internally visible serviceable indicia 80 can form a curvilinear pattern such that the pattern in the rear waist region 38 is concave relative to the longitudinal and transverse axes 100, 110 in order to match the contours of the wearer's buttocks. The pattern in the front waist region 36 also can be curvilinear relative to the longitudinal and transverse axes 100, 110 or else linear, but in either case, may if desired be contoured to match the sides of the wearer's lower back. As a result, the contoured pattern can be produced to provide an imprint of a wearer's lower back and buttocks region on the body-facing surface of the article directing the caregiver where to place the wearer during fitting. U.S. Patent Publication No. 2003/0158532 discloses various embodiments of internally visible serviceable.

The internally visible serviceable indicia 80 may be disposed on the body-facing surface of the topsheet 24 or beneath the topsheet 24 as long as it is visible from the body-facing surface. In addition, the internally visible serviceable indicia 80 can have different colors, color patterns, gradient patterns, or textures used in order to communicate softness of the article towards the body and also provide some opacity sufficient to mask any underlying layer or material, thereby preventing the visual discernment of the layer, material, or substance through the serviceable indicia.

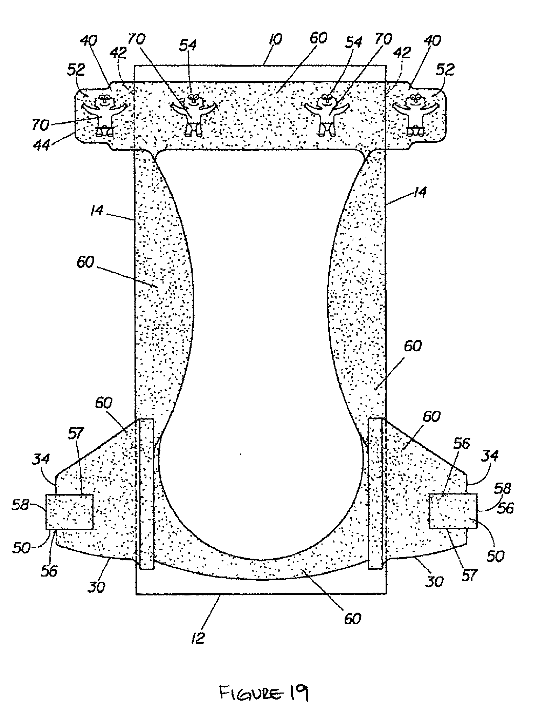

As previously described, the disposable absorbent article according to the present invention may include a fastening system 50 designed to facilitate an easy intuitive change. An example of an embodiment including such a fastening system 50 is shown in Figure 19. For this embodiment, the fastening system 50 releasably attaches the front waist region 36 to the rear waist region 38 and provides a first fit and a second fit. The first fit provides a loose fit enabling the article to be maneuvered about the wearer's lower torso during fitting and the second fit provides a secure fit about the wearer's waist. For instance, during the first fit, the diaper 20 may be fitted loosely around the wearer's ankles while standing and then pulled up around the wearer's waist and tightly secured by the second fit. The fastening system 50 includes primary and secondary landing members 52, 54 corresponding to the first fit and the second fit. In addition to curvature of the landing zone and a serviceable indicium 120 at the front waist region 36, the primary and secondary landing members 52, 54 (or a single landing zone 122) can include instructional serviceable indicium 70 providing instruction to the caregiver corresponding to the first fit and the second fit.

For this embodiment, the fastening system 50 may include a hook-and-loop-type fastener including at least one engaging component (male fastening component) and at least two landing zones (female fastening components). In this case, the two landing zones provide the primary and secondary landing members 52, 54. Alternatively, a single landing zone 122, as previously discussed, may be provided. Alternatively, the fastener may include a tab-and-slot-type fastener, wherein the tab member includes a retaining element that interlocks with an opening such as a slit, slot, or loop as disclosed in U.S. Patent No. 6,251,097. For this embodiment the tab-and-slot fastener comprises at least two slit, slot, or loop openings providing the primary and secondary landing members 52, 54. For tab-and-slot-type fasteners, primary and secondary landing members 52, 54 are separated by a distance. For hook-and-loop-type fasteners, the primary and secondary landing members 52, 54 may be separated by a distance or else contiguous.

The instructional serviceable indicia 70 disposed on the landing members include instructions designating matching connections between the tab members and first or second landing members 52, 54. The instructions might include graphics of characters illustrating the orientation and/or configuration of the diaper 20 during the first fit and the second fit. For instance, a graphic might illustrate a loose fitting diaper fitted about the waist of the character during the first fit and a tight fitting diaper secured about the waist of the character during the second fit.

Instructional serviceable indicia 70 disposed on the first and second landing members may be particularly useful where the diaper includes the versatility of being fitted to the wearer while the wearer is standing or lying down. For such an embodiment, the first fit may comprise fitting the diaper to the wearer while the wearer is standing in which case the diaper is fitted around the wearer's ankles by attaching the tabs to the first landing members and then pulled up around the lower torso of the wearer. Once the diaper is positioned around the lower torso, the diaper may be adjusted to achieve the second fit by removing the tabs from the first landing members and engaging them with the second landing members. For this embodiment, the graphic on the first landing member may include a character wearing a diaper around its ankles, while the graphic on the second landing member includes a character wearing a diaper secured about its waist.

In addition to graphics, the instructional serviceable indicia 70 may also comprise words such as "ankles" and "waist" associated with the position of the article during the first fit and the second fit, or they may designate the type of fit such as "loose" and "snug," or they may contain any other desired graphics or symbols. For example, the instructional marks may comprise numbers such as "1" and "2," designating the first and second fits, respectively.

In the embodiment shown in Figure 19, the fastening system 50 includes two tab members 56. Each tab member 56 has a tab proximal edge 57 disposed at the distal edge 34 of each of the rear ear panels 30, a tab distal edge 58, a tab body-facing surface, and a tab garment-facing surface. Each tab member 56 includes fastening elements disposed proximate the tab distal edge 58. Front ear panels 40 are disposed along each longitudinal side edge 14 in the front waist region 36. Each second ear panel has a body-facing surface and a garment-facing surface, a proximal edge 42 joined to the longitudinal side edge 14, and a distal edge 44 opposite the proximal edge 42. A primary landing member 52 is disposed on the garment-facing surface of each of the front ear panels 40. The secondary landing member 54 is disposed on the garment-facing surface of the article in the front waist region 36. During fastening, the primary landing member 52 provides a first fastening point, wherein once the tab member 56 is engaged a first fit is achieved. The first fit provides a loose fit about the wearer enabling the diaper to be maneuvered about to achieve an effective orientation for wear. The second landing member 54 provides a second fastening point for achieving a second fit where the article is tightly secured about the waist of the wearer. For this embodiment the instructional serviceable indicia comprise graphics. The graphic on the first landing member 52 includes a character wearing a diaper around its ankles while the graphic on the second landing member 54 includes a character wearing a diaper secured about its waist.

## Claims

1. A disposable absorbent article (20) to be worn about the lower torso of a wearer, the absorbent article comprising a topsheet (24), backsheet (26) and a core (28) disposed there between, having a front waist region, back waist region and a crotch region therein between, and comprising first and second front ear panels (40) extending outwardly from the front waist region, the absorbent article including at least one first externally visible serviceable indicium (120), proximate a front waist region (36) of the article, wherein the at least one externally visible first serviceable indicium has a curvature for alignment with the navel of the wearer, whereby the curvature is convex relative to a lateral axis of the article and whereby the at least one first externally visible serviceable indicium comprises a color, pattern or texture and whereby said article comprises further front ear panel serviceable indicia (160) provided on said first and second front ear panels (40).

2. A disposable absorbent article (20) as in claim 1, whereby said further front ear panel serviceable indicia (160) comprise at least a graphic (166).

3. A disposable absorbent article (20) as in claim 1 or 2, whereby said article comprises barrier leg cuffs and whereby the front ear panels (40) form at least a portion of the longitudinal edge of the article and are partially formed by the barrier leg cuffs.

4. A disposable absorbent article as in claim 1, whereby said further front ear panel serviceable indicia (160) span the entire front ear panels (40).

5. A disposable absorbent article (20) as in claim 1, comprising a landing zone (122) longitudinally spaced from the at least one first serviceable indicium (120).

6. A disposable absorbent article (20) as in claim 5, whereby said landing zone (122) comprises a second externally visible serviceable indicium and said second serviceable indicium comprising a color, pattern or texture.

7. The disposable absorbent article (20) of claim 6, whereby the landing zone comprises a curvature, the curvature forming the second serviceable indicium, preferably the curvature of the landing zone is complementary to the curvature of the first serviceable indicium, preferably the curvature of the landing zone is convex relative to a lateral axis of the disposable absorbent article.

8. The disposable absorbent article (20) of claim7, whereby the first serviceable indicium (120) has a top edge (128) and a bottom edge (130), the landing zone (122) has a top edge (132) and a bottom edge (134), and a first gap (124) is formed between the bottom edge of the first serviceable indicium and the top edge of the landing zone, preferably the landing zone has a length and wherein the first gap is approximately constant over at least about half the length of the landing zone, the first gap has a first gap width (324) of between 0.5 mm to 40 mm.

9. The disposable absorbent article (20) of claim 8, wherein the bottom edge of the first serviceable indicium and the top edge of the landing zone are parallel over at least about the two centimeter distance, preferably the bottom edge of the landing zone is generally parallel to the lateral axis of the disposable absorbent article, preferably the bottom edge of the landing zone is generally parallel to the top edge of the landing zone.

10. The disposable absorbent article (20) of claims 5 or 6, whereby second and third gaps (136) are provided between the landing zones (122) and the first and second ear panels, respectively, preferably the first, second and third gaps (124, 136) are generally continuous and form a composite gap.

11. The disposable absorbent article (20) of claim 1 further comprising first and second barrier leg cuffs (150), and said first and second front ear panels (40) are formed in part by the first and second barrier leg cuffs, respectively, wherein at least on the said further serviceable (160) indicia has a curvature, preferably the further front ear panel serviceable indicia (160) further comprise a color, pattern texture, or graphic.

12. The disposable absorbent article (20) of claim 7, wherein the first and second front ear panels (40) comprise a laterally inboard region (164) and a laterally outboard region (162) and wherein the laterally outboard region is formed by the barrier leg cuff (150), preferably the further front ear panels serviceable indicia (160) extend of their laterally outboard region of the first and second front ear panels, preferably the further front ear panel serviceable indicia (160) extend over the laterally outboard region and the laterally inboard region of the first and second ear panels.
